(19) Europäisches Patentamt
European Patent Office
Office européen des brevets

(11) Publication number: 0 367 734
A1

(12) EUROPEAN PATENT APPLICATION

(21) Application number: 89850290.1

(22) Date of filing: 06.09.89

(51) Int. Cl.[5] A61K 31/23 , A61K 9/10 , A23L 1/30 , A23K 1/16

(30) Priority: 07.09.88 SE 8803142

(43) Date of publication of application: 09.05.90 Bulletin 90/19

(84) Designated Contracting States: ES GR

(71) Applicant: KABIVITRUM AB

S-112 87 Stockholm(SE)

(72) Inventor: Lindgren, Svante
Lättsta Skepptuna
S-195 00 Märsta(SE)
Inventor: Jonsson, Uno
Tetorpsvägen 41
S-194 36 Upplands Väsby(SE)
Inventor: Sandberg, Göran
Björkskog
S-762 00 Rimbo(SE)
Inventor: Granelli, Kristina
Väderkvarnsgatan 8 B
S-753 29 Uppsala(SE)

(74) Representative: Onn, Thorsten et al
AB STOCKHOLMS PATENTBYRA Box 3129
S-103 62 Stockholm(SE)

# (54) Energy substrate containing hydroxycarboxylic acid and a glycerol ester.

(57) An energy substrate for administering nutrient to mammals in a catabolic state is characterised in that it contains at least one ester of glycerol and at least one hydroxy carboxylic acid having a branched carbon chain.

WEIGHT GAIN DURING TEST PERIOD

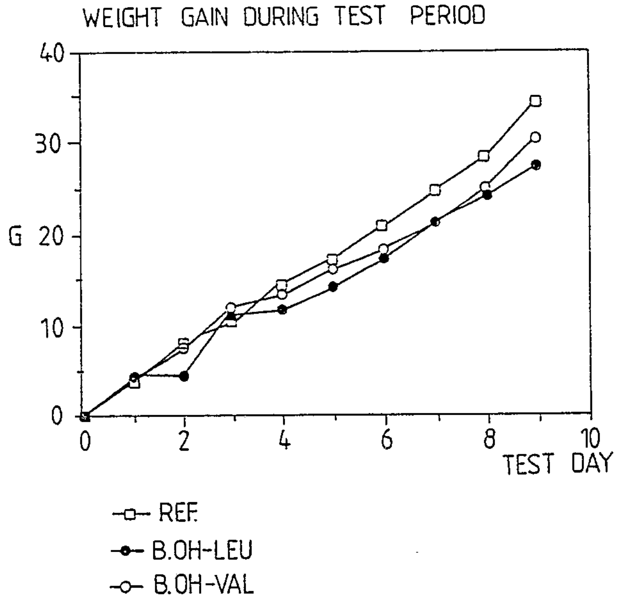


EP 0 367 734 A1

Xerox Copy Centre

# A new energy substrate

The present invention relates to pharmaceutical substrates and pertains to the field of clinical nutrition. This field is concerned with all forms of enteral and parenteral nutrition of mammals.

Those groups of patients subjected to clinical nutrition in its various forms often have a poor nutrition status. This status may be the cause of serious trauma, such as deep and extensive burns, surgical trauma, serious body injuries, various forms of cancer or sepsis. Other groups comprise patients who are unable to eat, for physiological, anatomical or other reasons, for instance unconciousness. In the case of serious trauma, however, the metabolic picture is complicated, because of the poor utilization of glucose in the peripheral tissues. Although the glucose and insulin levels of the blood increase, no energy, or relatively little energy is gained thereby. A so-called peripheral glucose intolerance with insulin resistance has arisen (Gump F.E., Long C., Killian P and Kinney J., M.,; J. TRAUMA 14(5): 378-388, 1974. Black P.R., Brooks D.C., Bessey P.Q., Wolfe R.R. and Wilmoore D.W. ANN.SURG. 196(4); 420-435, 1982 Drobney E.C., Abramson E.C. and Baumann G.J.CLIN. ENDOCRINOL.METAB. 58(4):710, 1984 etc.).

In the case of glucose intolerance, the oxidation of fat is also reduced and the ketone body metabolism is unchanged, which means that the total energy recovery is impaired (Ryan et al METAB. 23(11), 1081 1974., O'Donnel T.F., Blacburn G.L. "Proteolysis associated with a deficit of periferal energy fuel substrates in septic man. SURGERY 80; 192-200, 1972). In a situation such as this, the body mobilizes different body proteins as an energy source, which results in a more or less extreme protein catabolic situation.

By way of summary, the following metabolic conditions exist in, for instance, sepsis and also trauma conditions:

a) Increased proteolysis of muscle proteins and increased oxidation of amino acids.

b) Hyperglycemia. (The lever continues to produce glucose despite high insulin levels).

c) Increased peripheral take-up of glucose.

d) Increased production of pyruvate.

e) Reduced oxidation of pyruvate, as a result of restraint in the pyruvate metabolism.

f) Increased production of lactate, alanine and pyruvate.

g) Decreased sensitivity to the effect of insulin on the glucose metabolism in the muscle cells (insulin resistance).

h) A decrease in the plasma concentration of glutamine is also present in many trauma situations.

The aforementioned circumstances indicate the complicated metabolic conditions pertaining, inter alia, to the branched amino acids in stress situations. The net result is a disturbed energy conversion, where practically the only available substrate, branched amino acids, is converted to energy, although even then with certain limitations.

Many attempts have been made to normalize the clinical picture and to restore the concentration of branched amino acids in various stress conditions, but with varying results. It is necessary to observe certain matters, when giving nutrition parenterally to patients of this category.

1) The administration of solely glucose, intravenously, in the case of glucose intolerance has only negative effects in the main.

2) The administration of fat, intravenously, in the case of, e.g., sepsis, can in certain cases be directly contraindicative. A liver insufficiency namely exists with subsequent unchanged fat metabolism.

3) It is necessary to find a glucose substitute in the aforesaid stress situation with glucose intolerance.

4) The administration of branched amino acids gives varying results and can, in certain instances, be stressful in itself. Furthermore, oxidation of these amino acids results in an increase in the concentrations of nitrogen metabolites in the blood, a circumstance which must be strongly questioned in view of the fact that in the case of sepsis with, for instance, an endo-toxin shock with impaired liver function, the renal secretion of e.g., ammonia is impaired due to the lowered blood pressure, which must be considered as highly toxic to the brain.

5) A possible, new substrate should be capable of replacing amino acids, glucose and fat from an energy aspect, even under normal metabolic conditions.

In in-vitro experiments with rat liver cells, the decarboxylation rate was stimulated by branched $\alpha$-keto acids to an extent which was from 2 to 9 times faster than the stimulation rate achieved with corresponding branched amino acids. An advantage is gained when branched keto acids can replace partially, e.g., glucose. No experiments with branched keto acids as an energy-boost source have previously been carried out. Instead, trials with branched keto acids have been intended for the administration of nitrogen-free

amino acid substitutes to, for instance, uremics and those suffering from liver diseases (DE 2335215, 2335216, US Patent Specification 4,100,161, 4,100,160, EP 0 227 545). It has been difficult, however, in practice, to produce and store solutions which contain branched α-keto acids in free form, since such acids have an extremely poor stability. Acids bound to different carriers, such as metals, alkali metals and alkaline-earth metals have also shown poor stability. One solution (e.g. proposed in DE 2335215) to the problem of the poor stability and durability of the keto acids in storage is to use a concentrated solution which has been sterile-filtered and frozen. Prior to use, the keto-acid concentrate is thawed and diluted to the correct concentration. The significantly more durable branched α-hydroxy acid homologues have also been used. These have been used, however, solely as nitrogen-free amino-acid substitutes (German Patent Speification 2335215 and 2335216). By branched hydroxy acids is meant in the present description and claims branched amino acids (leucine, isoleucine and valine) these amino groups being substituted with hydroxy groups. The resultant acids can be converted to the respective α-keto acids, through specific enzymatic reactions. The enzymes taking part in these conversions are found generally in various organs of the majority of mammals, among them human beings.

The following general relationship can be written:

$$R - \underset{NH_2}{\underset{|}{CH}} - COOH \rightleftharpoons R - \underset{O}{C} - COOH \rightleftharpoons R - \underset{OH}{\underset{|}{CH}} - COOH$$

where R may be an aliphatic, branched or aromatic group. Branched hydroxy acids normally have an L-form, although certain D-forms can be used by some mammal species (Close J, H, New Engl J Med, 22 March, pages 663-667, 1974). Experiments on branched hydroxy acids as amino-acid substitutes have shown that they are able to form the respective amino acids (how K W and Walser M, J Nutr 105:372, 378, 1975; Pond W G et al, J Nutr 83:85-93, 1964; Chawla R K and Rudman D.J., J Clin Invest 54:271-277, 1974, etc). Thus, the ability of branched hydroxy acids to convert to respective keto acids has been shown by several authors. The present invention utilizes this fact, insomuch as the resultant keto acid is included directly as a substrate in the oxidation of branched amino acids, by excluding the transamination step. The metabolic path of α-keto leucine will now be described:

α-keto leucine → isovaleryl-CoA → ß-crotonyl-CoA
irrev.
→ ß-methyl-glutaconyl-CoA → citrate cycle → the breathing chain.

The end products of α-keto leucine are acetyl-CoA and propionyl-CoA. This last mentioned is converted to succinyl-CoA, which is also an end product for α-keto valine. Succinyl-CoA is already present in the citrate cycle and also in the breathing chain for energy recovery ($NADH + H^+$ and $FADH_2$). Branched α-hydroxy acid can be used in the aforedescribed manner as a substitute energy substrate for branched α-keto acids.

Another possibility for branched α-hydroxy acids to generate energy, is to pass directly through the inner mitochondria membrane and into the matrix, where they are converted enzymatically (via a lactate dehydrogenase) to the respective α-keto acid. The reaction can be described as a shuttle in accordance with the following:

Branched α-hydroxy acid ⇄ branched α-keto acid
$NAD^+$ $NADH^+H^+$

The system is described for the spermatozoa of mice (Gerez de Burgos et al, Biochem Biphys Res Comm 81(2): 644-649, 1978; Burgos C et al, Biochem J 208:413-417, 1982). This system is thought to be capable of generating energy, partly through oxidation of the α-keto acid formed and partly through the delivery of cytosol-protons, which are able to enter the breathing chain directly as $NADH^+H^+$.

One drawback with the use of pure acids, however, is that, from a molar aspect, they normally require

equivalent quantities of cations in order for the solutions to obtain a physiologically adapted pH. These cations may, for instance, be sodium, potassium, calcium or magnesium. Each type of ion may be present separately or in mixture with one or more of the others. The admixture of electrolytes may, however, result in excessively high levels of osmolality in the infusion solution, which increases the risk of chemical irritation of the vein in which the infusion is made (the risk of thrombo-phlebitis increases). This means that infusion of such a solution should only be effected in a large, central vessel, via a central vein catheter. The surgical implantation of such catheters is more difficult, more risky and more expensive than the insertion of a so-called peripheral catheter. Furthermore, it is necessary to restrict the supply of calcium and magnesium, due to the risk of precipitation and subsequent blocking of the catheter. It must be considered particularly riskful to supply large quantities of electrolytes to a patient suffering from shock after, for instance, sepsis, with reduced renal excretion, inter alia, from the aspect of circulation. Neither is it possible to exclude a certain negative influence on transport processes out and into the mitochondria.

Water-soluble monoglycerides of different fatty acids having short chains and average chains have been produced and tested in different animal models (Birkhahn R H et al, New synthetic substrates for parenteral feeding. J Parenteral and Enteral Nutrition, vil. 3, nr 5 , 1979). Different esterifications between different fatty acids (oxo- and hydroxy-) and different alcohols, among them glycerols, have also been described in US Pat. 4,665,057.

The aforementioned drawbacks are avoided by the present invention, which enables large quantities of the branched hydroxy acids to be administered without needing to administer large quantities of electrolytes. This is achieved in accordance with the present invention, by administering the hydroxy acids in the form of esters with an alcohol, and then particulary glycerol.

Accordingly, the present invention relates to an energy substrate for nutrient supply to a mammal in a catabolic state, and is characterized in that the energy substrate contains at least one ester of glycerol and a hydroxy carboxylic acid having a branched carbon chain.

The branched hydroxy carboxylic acid has particularly the general formula

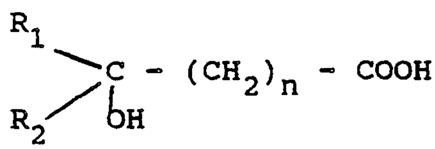

where $R_1$ is hydrogen or straight or branched alkyl or alkenyl, and $R_2$ is straight or branched alkyl or alkenyl, wherein where $R_1$ is hydrogen, $R_2$ is always branched alkyl or alkenyl, $R_1$ and $R_2$ together contain from 2 to 6 carbon atoms, and n is zero or 1.

The value of n in the formula is preferably zero, so that the hydroxy group will always sit in the $\alpha$-position of the carboxyl group. The hydroxy group, however, may also sit on the $\beta$-position, in which case n is 1.

In the case of one preferred embodiment, the hydroxy carboxylic acid comprises at least one of the acids L-$\alpha$-hydroxy-isocaproic acid, $\alpha$-hydroxy-$\beta$-methyl valeric acid and L-$\alpha$-hydroxy-isovaleric acid.

One, two or three of the hydroxy groups in the glycerol molecule may be esterified with hydroxy carboxylic acid. Preferably, one or two of the hydroxy groups will be esterified with hydroxy carboxylic acid, however, wherein the remaining hydroxy group or groups is/are esterified with one or more fatty acids having from 4 to 22 carbon atoms in its/their carbon chains.

The glycerol ester or esters may be derived from glycerides in a pharmaceutically acceptable fat which has been transesterified with one or more of the hydroxy carboxylic acids.

The glycerol esters used in accordance with the invention may be prepared by pure organic synthesis or semi-synthetically, by chemical transesterification of a natulral triglyceride having one branched hydroxy carboxylic acid. Transesterification can also be effected biochemically, with the aid of an enzyme system together with triglyceride and branched hydroxy carboxylic acid.

A semi-synthetic transesterification can be effected, for instance, on the basis of a vegetable or animal oil and a branched hydroxy carboxylic acid, and with the use of a catalyst, such as sodium ethoxide.

A purely organic-chemical synthesis will often result in uniform esters, which also applies to polyol esters. Statistically, uniform esters are also obtained when transesterifying with sodium ethoxide. An enzymatic transesterification, on the other hand, is often position-specific, for instance then for the positions 1 and 3.

From a biological aspect, it can be expected that uniform triglycerides will be more toxic than non-uniform triglycerides. It is therefore desirable to prepare so-called structurized lipids, where only one or two of the hydroxy groups of the glycerol are esterified with divergent aliphatic fatty acids, for instance branched hydroxy carboxylic acids. The remaining hydroxy groups, i.e. one or two such groups, are then esterified with "normal" fatty acids having a carbon chain of average lengths or long carbon chains.

One method of obtaining the desired structure when transesterifying, is to use various lipases with additions of the fatty acid to be incorporated. An example of one such enzyme is Lipozyme[R], available commercially from Novo Industrier, Copenhagen, Denmark. This enzyme, which is available in an immobilized form, so that it can be used repeatedly, is of microbiological origin (Mucor miehei), and is specifically lipolytic in positions 1 and 3 of the triglyceride.

Naturally, the vegetable or animal oil or fat used as the starting material in the transesterification process must be pharmaceutically acceptable. Examples of suitable oils in this connection are soya oil, sunflower oil, Oenothera bie'nnis oil, oil from Borago (Borage), or blackcurrent oil and fish oils.

Energy substrates produced in accordance with the invention will suitably contain between 1 and 100 percent by weight of esterified hydroxy carboxylic acid, and then preferably between 20 and 60 percent by weight.

In order to administer a fat intravenously, it is necessary to predisperse the fat to form an emulsion with the aid of a suitable emulsifier. Such an emulsion may comprise, for instance, 0.1-50% w/w in water of a pharmaceutically acceptable oil or fat, 0.1-6% w/w of phospholipids prepared from eggs or soya beans, 0.1-4.5% w/w of glycerol or some other isotonic substance, and for instance a carbohydrate such as glucose or fructose. Phosphatidyl glycerol and/or phosphatidic acid, fatty acids (8-22 carbon atoms), and alkali salts of fatty acids, for instance Na-oleate may also be included in a total concentration of 0.1-5% as additional emulsion stabilizers.

Emulsions for use as a nutrient substrate in accordance with the present invention are prepared in accordance with conventional pharmaceutic practice, in a manner well known to the person skilled in this art. In this respect, it is essential that the constituents used are of a pharmaceutically non-objectionable character. For instance, it is necessary to protect the triglycerides and emulsifiers used against oxidation, for example by storing the glycerides and emulsifiers under the protection of a protective gas, e.g. nitrogen gas, and the resultant emulsion should also be stored in a protective-gas environment. When the emulsion is administered parenterally, e.g. intravenously, the water used must also be sterile and free of pyrogens.

The emulsions prepared in accordance with the invention may also contain additional nutrients, such as amino acids, carbohydrates and other fat substances of vegetable, animal or synthetic origin. The emulsions may also contain vitamins, trace elements and salts. This will enable the triglycerides used in accordance with the invention to be included in a system which will allow a complete nutrient supply to be administered enterally or parenterally.

When the inventive emulsions are to be administered parenterally, the emulsions will preferably have an average particle size of beneath 0.5 micron. A manner of achieving this is described in the following examples (Nos. 10 and 11). Example 11 also illustrates how mammals are able to utilize the inventive triglycerides biologically as energy substrates instead of glucose and fat from a conventional fat emulsion.

The invention will now be illustrated further with reference to a number of examples, although it will be understood that these examples have no limitive significance. All concentrations are based on the final weight of an ultimate emulsion.

The single figure of the accompany drawing is a comparison diagram which compares the increase in weight of a group of rats administered with nutrient in the form of an energy substrate prepared in accordance with the invention, with the weight increase of a control group to which conventional nutrient was administered parenterally.

## Example 1

26.55 g L-α-hydroxy leucine was dissolved in 88.4 g triolein in a nitrogen-gas atmosphere at +65 °C, while stirring the system. The solution was admixed with 0.8 ml distilled water and 11.5 g Lipozyme® Novo (Batch 1739), an immobilized lipase which is active solely on the 1-position and 3-position on triglycerides. The reaction then continued for 1 calender day, after which the enzyme was filtered off and the crude product dissolved in cold petroleum ether (888 ml), wherewith non-reacted L-α-hydroxy leucine precipitated out. This precipitate was filtered from the petroleum ether solution. A further 612 ml of petroleum ether were added to the petroleum ether solution, and the solution was allowed to stand at -20 °C for one calender day. The precipitate formed herewith was filtered off and weighed and the filtrate evaporated under vacuum conditions to a constant weight. Analysis of the precipitate showed that 38.4% of the L-α-hydroxy leucine

charged to the system was present in a chemically bound form. When recalculated to new triglyceride. the L-α-hydroxy leucine was found to be 29.4% of the total triglyceride content.

Example 2

26.55 g of L-α-hydroxy leucine were dissolved in 88.9 g triolein in a nitrogen-gas atmosphere at +65 °C, while stirring the system. 0.8 ml of distilled water and 11.55 g of Lipozyme[R] were added to the solution. The reaction continued over 4 calender days, during which a further 0.8 ml of distilled water was added to the solution on day 2 and day 4. At the end of the fourth day period, the enzyme was filtered-off and the crude product dissolved in cold petroleum ether (875 ml) and stored at -20 °C for 1 calender day. The resultant precipitate of free L-α-hydroxy leucine was then filtered-off and the filtrate evaporated under vacuum conditions to constant weight. The precipitate was then extracted in distilled water twice (400 ml), in order to isolate the final residues of hydroxy leucine. Analysis of the precipitate showed that 49.5% of the L-α-hydroxy leucine introduced to the system was present in a chemically bound form, and when recalculated to new triglyceride was found to comprise 38.4% of residual triglycerides.

Example 3

53.1 g of L-α-hydroxy leucine were dissolved in 88.9 g triolein in a nitrogen-gas atmosphere at +65 °C. while stirring the system. 0.7 ml of distilled water and 14.2 g of Lipozyme[R] were added to the solution. The reaction continued for 4 calender days, during which a further 0.4 ml of distilled water was added to the solution on day 2 and day 3. At the end of the 4-day period, the enzyme was filtered-off and the crude product dissolved in 2500 ml of cold petroleum ether. The solution was allowed to stand for 1 calender day under cold conditions. The resultant precipitate of free α-hydroxy leucine was then filtered off and the filtrate evaporated under vacuum conditions to constant weight. The filtrate was then extracted 10 times with distilled water (10) x 500 ml), in order to extract the final residues of free L-α-hydroxy leucine. Analysis of the precipitate showed that 69% of the stoichiometric quantity of L-α-hydroxy leucine had bound chemically, which when recalculated to new triglyceride was found to be 60.2% of the residual triglycerides.

Example 4

47.5 g of L-α-hydroxy valine were dissolved in 89.0 g of triolein in a nitrogen-gas atmosphere at +75 °C, while stirring the system. 0.8 ml of distilled water at 13.7 g of Lipozyme[R] was added to the solution. The reaction continued for 5 calender days, during which a further 0.4 ml of distilled water was added to the solution during day 2. At the end of the 5 calender day period. the enzyme was filtered off and the crude product dissolved in 2500 ml of cold petroleum ether. and the solution was allowed to stand for one calender day in cold conditions. The resultant precipitate of free L-α-hydroxy valine was then filtered-off and the filtrate evaporated under vacuum conditions to constant weight. The filtrate was then extracted with distilled water (10 x 500 ml), in order to extract the final residues of free L-α-hydroxy valine. Analysis of the precipitate showed that 51.6% of the stoichiometric quantity of L-α-hydroxy valine had bound chemically, which when recalculated to new triglyceride was found to be 40.6% of residual triglycerides.

Example 5

55.0 g of L-α-hydroxy leucine were dissolved in 90.3 g of refined soybean oil, in a nitrogen-gas atmosphere at 75 °C, while stirring the system. 0.8 ml of distilled water and 14.5 g of Lipozyme® were added to the solution. The reaction continued for 4 calender days, during which a further 0.4 ml of distilled water was added during day 3. At the end of the 4-day period, the enzyme was filtered off and the crude product dissolved in 2500 ml of cold petroleum ether, and the solution was allowed to stand for 1 calender day in cold conditions. No precipitation was observed. The solution was then evaporated to constant weight and extracted with distilled water (10 x 500 ml), in order to isolate free L-α-hydroxy leucine. Analysis of the evaporation residue showed that about 100% of the stoichiometric quantity of L-α-hydroxy leucine had bound chemically, which when calculated to new triglyceride was also about 100%.

Example 6

265 g of L-α-hydroxy valine were dissolved in 500 g of pure soybean oil, in a nitrogen-gas atmosphere at +65 °C, while stirring the system. The reaction continued for 4 calender days, during which 1 ml of distilled water was added to the solution on days 2, 3 and 4. At the end of the 4-day period, the enzyme was filtered off and the crude product dissolved in 5000 ml of cold petroleum ether. The solution was allowed to stand for 1 calender day in cold conditions. The resultant precipitate of free L-α-hydroxy valine was then filtered off and the filtrate evaporated under vacuum conditions to constant weight. The resultant oil was decolourized with the aid of a known method using 10% fullers' earth and 1% activated carbon. The oil was then treated with soda in accordance with known techniques, in order to extract free fatty acids. The oil was finally dried with dry sodium sulphate. Upon completion of this stage, the amount of free fatty acids was measured as 1.1 mmol/kg. The yield, calculated on the incorporated amount of L-α- hydroxy valine, was found to be 62.0% of the stoichiometric quantity and, when calculated to new triglyceride, was found to be 50.74% of the total amount of triglycerides.

Example 7

265 g of L-α-hydroxy valine were dissolved in 500 g of refined soybean oil in a nitrogen-gas atmosphere. at +62 °C, while stirring the system. 4 ml of distilled water and 76.5 g of Lipozyme[R] were added to the solution. The reaction continued for 4 calender days, during which a further 2 ml of distilled water were added to the solution on days 2, 3 and 4. The enzyme was then filtered off and the crude product dissolved in 5000 ml of cold petroleum ether, and the solution was allowed to stand for 1 calender day, in cold conditions. The resultant precipitate of free L-α-hydroxy valine was then filtered off and the filtrate obtained was evaporated under vacuum conditions to constant weight. The resultant oil was decolourized with the aid of a known method, using 20% fullers' earth and 1% activated carbon. The oil was then treated with soda in accordance with a known technique, in order to isolate free fatty acids. The oil was finally dried with dry sodium sulphate. Upon completion of this stage, the amount of free fatty acids present was measured as 5.5 mmol/kg. The yield calculated on the incorporated amount of L-α-hydroxy valine was 47.6% of the stoichiometric quantity, which when recalculated to new triglyceride was found to comprise 36.4% of the total amount of triglycerides.

Example 8

305 g of L-α-hydroxy leucine were dissolved in 500 g of refined soybean oil in a nitrogen-gas atmosphere at +65 °C, while stirring the system. 4 ml of distilled water and 80.5 g of LipozymeR were added to the solution. The reaction continued for 4 calender days, during which a further 2 ml of distilled water were added to the solution on days 2, 3 and 4. The enzyme was then filtered off and the crude product decolourized with the aid of a known method, using 10% fullers' earth and 1% activated carbon. The resultant oil was treated with soda in a known manner, in order to remove free fatty acids. The oil was finally dried with dry sodium sulphate. At this stage, the amount of free fatty acids present was measured as 4.2 mmol/kg. The yield calculated on the incorporated amount of L-α-hydroxy leucine was found to be 88.96% of the stoichiometric quantity, which when recalculated to new triglyceride was found to be 84.25% of the total amount of triglycerides.

Example 9

Oil on which L-α-hydroxy leucine was bound and which was prepared in accordance with the processes described in the previous examples was mixed with refined soybean oil in the proportions of 114.7/85.3. The mixture was sterile-filtered with the aid of a sterile filter 0.45 μm of the type Millipore[R]. 30.0 g of egg-yolk phospholipides were dissolved in 400 ml of the aforesaid sterile oil, at 60 °C. 88.8 g of glycerol (50% in water) were dissolved in about 1477 ml of heated distilled water (+80 °C). The oil-phospholipid solution was preemulsified with the aid of a Janke-Kunkel Ultra-Turrax over a period of 10 minutes, wherein 4.0 ml of a 1 M aqueous solution of sodium hydroxide were also added. The resultant precursory emulsion was finally emulsified in a valve-homogenizer of the type Manton-Gaulin M-2, in accordance with conventional technique. The final emulsion was dispensed into glass flasks or bottles under vacuum and in a nitrogen-gas atmosphere, whereafter the flasks were sealed with rubber stoppers and capsules. The emulsion was then heat-sterilized in accordance with an accepted technique.

## Example 10

30 g of egg-yolk phospholipids were dissolved in 400 g of oil having L-α-hydroxy valine bound thereto and produced in accordance with the process described in Example 7. 88.8 g of glycerol (50% in water) were dissolved in hot (+80 °C) distilled water (ca 1477 ml). The oil-phospholipid solution was preemulsified with the aid of a Janke-Kunkel Ultra-Turrax over a period of 10 minutes, during which 4.0 ml of 1 M sodium hydroxide solution were added. The resultant precursory emulsion was finally emulsified conventionally in a valve homogenizer of type Manton-Gaulin M-2. The resultant emulsion was dispensed into glass flasks or bottles, under vacuum conditions and in a nitrogen-gas atmosphere, and the flasks sealed with rubber stoppers and capsules. The emulsion was then heat-sterilized in accordance with an accepted technique.

## Example 11

A central vein catheter was implanted surgically into male rats of the strain Sprague-Dawley, weighing 180-190 g. The catheters were protected by a saddle (Roos et al, 1981). Nutrients were administered parenterally and comprised the following standard program or formula:

| | |
|---|---|
| Fat | 8 g/kg body weight and 24 hours |
| Nitrogen | 1.2 g/kg body weight and 24 hours |
| Total energy supplied | 1257 kJ/kg body weight and 24 hours |
| NPC (non-protein calories) | 1098 kJ/kg body weight and 24 hours |
| Liquid | 300 ml/kg body weight and 24 hours |

The ratio between glucose and fat was 70/30 energy percent. The energy percentage from the energy substitute during the test period was about 15% of NPC. The TPN-mixture was obtained by mixing Vamin$^R$ 14 (a solution of essential and non-essential amino acids for parenteral nutrient administration; electrolyte free), glucose solution 50% and Intralipid$^R$ 20% in suitable proportions, with the addition of adequate quantities of electrolytes, trace elements and vitamins.

Subsequent to continuous infusion over a period of one week (20 hours per day), the rats were divided randomly into 3 groups, with 6 animals in each group. One group was treated with the same TPN-program as earlier (reference group). The other groups (the test groups) were administered with L-α-hydroxy leucine and L-α-hydroxy valine in bound form respectively (prepared in accordance with the process described, inter alia, in Examples 6, 7, 8) as new fat emulsion (Examples 9 and 10). The amount of bound, branched hydroxy acid was administered in replacement of an equivalent amount of glucose energy. In other respects, the TPN-program was the same as that used for the reference group. The amount of bound L-α-hydroxy leucine administered equalled 2.0 g/kg body weight and day, while the amount of L-α-hydroxy valine administered corresponded to 1.8 g/kg body weight and day. This corresponds respectively to 4.5 g and 4.3 g of new triglyceride per kg of body weight and day, with L-α-hydroxy leucine and L-α-hydroxy valine in the 1 and 3 positions respectively of a structurized lipid. In total, the animals received daily 8-10 g of fat for each kg of body weight. About half the fat dosage consisted of new triglyceride. The animal experi ment then continued for 9 consecutive calender days, during which the weight of the animals was recorded daily and the urine secreted was collected and frozen. At the end of the test period, the rats were killed, by heart puncture while under a pentobarbital anesthetic. An autopsy was then performed with microscopic examination. The body organs were sent to a laboratory for pathological assay.

No difference in weight was observed between the three groups (Figure 1). The discretion of urine-urea-nitrogen in mg/kg and day during the test periods revealed no differences between the groups (Table 1). The relative organ weights, dry contents, dry weights, carcass fat contents, fat-free dry weight and fat weight revealed no deviations significant from a biological aspect (Tables 2-7). Neither did blood-chemical analyses (Table 8) show deviations from the reference group of biological significance. The pathological assay carried out on the body organs showed that no differences existed between the body organs taken from the test groups and the body organs from the reference groups, and that those small differences which

could be observed were to be found in all animals subjected to total parenteral nutrition including fat in emulsion form. In summary, the results obtained indicate that the novel triglycerides containing bound L-α-hydroxy leucine and L-α-hydroxy valine respectively are well tolerated and utilized as energy in those dosages administered.

Table 1

| Average secretion of urea-nitrogen during the test period | | | |
|---|---|---|---|
| | Reference group | Test group (OH-Leu) | Test group (OH-Val) |
| Mg/24 hour | 306.23 | 320.97 | 312.81 |
| S.D. | 76.98 | 94.67 | 73.15 |
| n* | 45 | 54 | 54 |

* n = the number of test occasions (number of rats in each group x number of test days)

Table 2

| Relative body-organ weights after the test period | | | | | | |
|---|---|---|---|---|---|---|
| | Reference group | | Test group (OH-Leu) | | Test group (OH-Val) | |
| | X | S.D. | X | S.D. | X | S.D. |
| Liver g/kg | 32.40 | 4.13 | 38.20 | 1.64 | 36.62 | 3.29 |
| Spleen g/kg | 2.76 | 0.22 | 3.79* | 0.45 | 3.37* | 0.33 |
| Kidneys g/kg | 7.85 | 0.20 | 8.40 | 0.41 | 8.09 | 0.43 |
| Lungs g/kg | 4.32 | 0.38 | 4.59 | 0.15 | 4.56 | 0.25 |
| Heart g/kg | 3.86 | 0.16 | 4.08 | 0.11 | 4.10 | 0.14 |
| Thymus g/kg | 2.52 | 0.13 | 2.47 | 0.08 | 2.53 | 0.36 |
| n | 5 | | 6 | | 6 | |

* $P<0.05$

Table 3

| Dry solid content of some body-organs after the test period | | | | | | |
|---|---|---|---|---|---|---|
| | Reference group | | Test group (OH-Leu) | | Test group (OH-Val) | |
| | X | S.D. | X | S.D. | X | S.D. |
| EDL-muscle(%) | 24.78 | 1.06 | 25.42 | 0.65 | 24.59 | 2.12 |
| Liver(%) | 32.49 | 1.13 | 35.95 | 1.78 | 35.41 | 0.53 |
| Kidneys(%) | 25.98 | 1.34 | 24.97 | 1.39 | 25.5 | 1.24 |
| Heart(%) | 24.65 | 1.16 | 23.80 | 1.35 | 24.06 | 0.57 |
| Carcass(%) | 39.62 | 0.79 | 40.11 | 0.82 | 38.57 | 1.9 |
| n | 5 | | 6 | | 6 | |

Table 4

| Dry solid weight of some body-organs after the test period | | | | | | |
|---|---|---|---|---|---|---|
| | Reference group | | Test group (OH-Leu) | | Test group (OH-Val) | |
| | X | S.D. | X | S.D. | X | S.D. |
| EDL-muscle(mg) | 26.78 | 2.74 | 26.47 | 1.23 | 26.59 | 2.25 |
| Liver (g) | 2.42 | 0.54 | 2.95 | 0.24 | 2.78 | 0.26 |
| Kidneys(g) | 0.46 | 0.04 | 0.45 | 0.04 | 0.45 | 0.03 |
| Heart(g) | 0.22 | 0.02 | 0.21 | 0.02 | 0.22 | 0.01 |
| Carcass(g) | 80.30 | 8.02 | 76.30 | 4.23 | 75.00 | 4.42 |
| n | 5 | | 6 | | 6 | |

Table 5

| Fat content of freeze-dried carcass after the test period | | | | | | |
|---|---|---|---|---|---|---|
| | Reference group | | Test group (OH-Leu) | | Test group (OH-Val) | |
| | X | S.D. | X | S.D. | X | S.D. |
| % | 34.84 | 1.50 | 34.62 | 2.08 | 31.76 | 4.89 |
| n | 5 | | 6 | | 6 | |

Table 6

| Fat-free dry weight of freeze-dried carcass | | | |
|---|---|---|---|
| | Reference group | Test group (OH-Leu) | Test group (OH-Val |
| G | 48.45 | 45.70 | 47.22 |
| S.D. | 4.10 | 2.26 | 2.33 |
| n | 5 | 6 | 6 |

Table 7

| Fat weight of freeze-dried carcass | | | |
|---|---|---|---|
| | Reference group | Test group (OH-Leu) | Test group (OH-Val |
| G | 27.11 | 26.10 | 23.62 |
| S.D. | 3.59 | 2.79 | 4.72 |
| n | 5 | 6 | 6 |

Table 8

| Blood-chemical analyses after test period | | | | | | |
|---|---|---|---|---|---|---|
| | Reference group | | Test group (OH-Leu) | | Test group (OH-Val) | |
| | X | S.D. | X | S.D. | X | S.D. |
| ASAT (µkat 1) | 1.56 | 0.16 | 1.63 | 0.15 | 1.53 | 0.15 |
| ALAT (µkat/1) | 0.47 | 0.53 | 0.41 | 0.07 | 0.37 | 0.09 |
| AP (µkat/1) | 5.17 | 0.53 | 4.01* | 0.43 | 3.97* | 0.37 |
| LD (µkat/1) | 21.71 | 6.48 | 21.57 | 6.46 | 24.85 | 6.7 |
| Bilirubin (µkat/1) | 3.60 | 0.60 | 4.70* | 0.50 | 3.20 | 0.40 |
| n | 5 | | 6 | | 6 | |

* $P<0.05$

## Claims

1. An energy substrate for administering nutrient to a mammal in a catabolic state, characterised in that the energy substrate includes at least one ester of glycerol and at least one hydroxy carboxylic acid having a branched carbon chain.

2. An energy substrate according to Claim 1, characterised in that the hydroxy carboxylic acid has the general formula

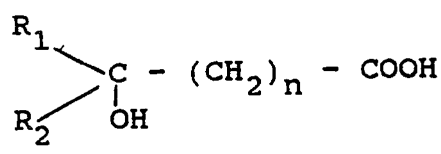

where $R_1$ is hydrogen or straight or branched alkyl or alkenyl, and $R_2$ is straight or branched alkyl or alkenyl, wherein when $R_1$ is hydrogen, $R_2$ is always branched alkyl or alkenyl, $R_1$ and $R_2$ together contain from 2 to 6 carbon atoms, and n is either zero or 1.

3. An energy substrate according to Claim 2, characterised in that n in the formula is zero.

4. An energy substrate according to any one of Claims 1-3, characterised in that the hydroxy carboxylic acid comprises at least one of the acids L-α-hydroxy-isocapronic acid, α-hydroxy-β-methylvaleric acid and L-α-hydroxy-isovaleric acid.

5. An energy substrate according to any one of Claims 1-3, characterised in that one or two of the hydroxyl groups of the glycerol are esterified with hydroxy carboxylic acid, whereas the remaining hydroxyl

group or groups in the glycerol is or are esterified with one or more fatty acids having 4-22 carbon atoms in their carbon chains.

6. An energy substrate according to any one of Claims 1-5, characterised in that the glycerol ester or esters is or are derived from glycerides in a pharmaceutically acceptable fat or oil which has been transesterified with one or more of the hydroxy carboxylic acids.

7. An energy substrate according to any one of Claims 1-6, characterised in that the energy substrate contains between 1 and 100 percent by weight of esterified hydroxy carboxylic acid, and then preferably 20-60 percent by weight.

8. An energy substrate according to any one of Claims 1-7, characterised in that the ester or esters exists, or exist, in the form of an emulsion in an aqueous phase; and in that the energy substrate further includes one or more of the components: emulsifier of vegetable, animal or synthetic origin, stabilizer, isotonic agent, vitamins, electrolytes and trace elements.

9. An energy substrate according to Claim 8, characterised in that the emulsifier consists of egg-yolk phospholipids or soya phospholipids or a mixture or fraction thereof.

10. An energy substrate according to Claims 8 or 9, characterised in that the isotonic agent is glycerol.

11. An energy substrate according to any one of Claims 1-10, characterised in that the ester or esters is, or are, dissolved in a pharmaceutically acceptable fat or oil.

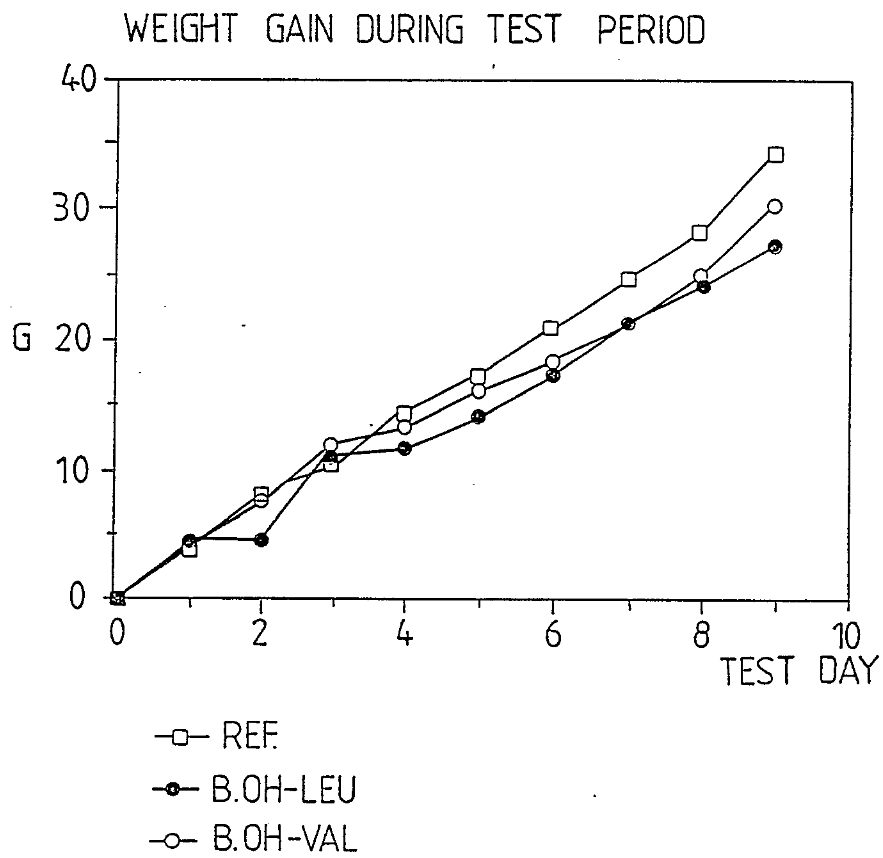
WEIGHT GAIN DURING TEST PERIOD
40
30
G 20
10
0
0
2
4
6
8
10
TEST DAY
REF.
B.OH-LEU
B.OH-VAL

European Patent Office

# EUROPEAN SEARCH REPORT

Application number

EP 89 85 0290.1

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| Y | WO-A1-85/03002 (BAXTER TRAVENOL LABORATORIES)<br>--- | 1-11 | A 61 K 31/23, 9/10<br>A 23 L 1/30<br>A 23 K 1/16 |
| Y | WO-A1-84/03625 (BAXTER TRAVENOL LABORATORIES)<br>--- | 1-11 | |
| Y | The Journal of Clinical Investigation, Vol 54, August 1974, Rajender K. Chawla and Daniel Rudman: "Utilization of x-Keto and Hydroxy Analogues of Valine by the Growing Rat", *Pages 271-277*<br>--- | 1-11 | |
| Y | J. Nutrition, Vol 83, 1964, W.G. Pond et al: "Effects of the x-Hydroxy Analogues of Isoleucine, Lysine, Threonine and Tryptophan and the x-Keto Analogue of Tryptophan and the Level of the Corresponding Amino Acids on Growth of Rats", *Pages 85-93*<br>--- | 1-11 | TECHNICAL FIELDS SEARCHED (Int. Cl.4)<br>A 61 K<br>A 23 L<br>A 23 K |
| Y | Journal of nutrition, Vol 105, 1975 Kye-Wing Chow and Mackenzie Walser: "Effects of Substitution of Methionine, Leucine, Phenylalanine, or Valine by Their x-Hydroxy Analogs in the Diet of Rats" *Pages 372-378*<br>------ | 1-11 | |

The present search report has been drawn up for all claims

| Place of search | Date of completion of the search | Examiner |
|---|---|---|
| STOCKHOLM | 14-11-1989 | SIÖSTEEN Y |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document